# EUROPEAN PATENT SPECIFICATION

(11) **EP 2 986 206 B1**
(45) Date of publication and mention of the grant of the patent: **05.12.2018**
(21) Application number: 14758753.9
(22) Date of filing: 18.08.2014
(51) Int. Cl.: A61B 5/042, A61B 5/00

(54) **SYSTEM FOR GENERATING ELECTROPHYSIOLOGY MAPS**
SYSTEM ZUR ERZEUGUNG VON ELEKTROPHYSIOLOGISCHEN KARTEN
SYSTÈME POUR GÉNÉRER DES CARTES D'ÉLECTROPHYSIOLOGIE

(30) Priority: 20.08.2013 US 201361867860 P
(43) Date of publication of application: 24.02.2016
(73) Proprietor: St. Jude Medical Atrial Fibrillation Division Inc., St. Paul, MN 55117 (US)
(72) Inventor: KOYRAKH, Lev A., Plymouth, Minnesota 55442 (US); HAGFORS, Mark, North Oaks, Minnesota 55127 (US); PRANAITIS, Simon T., Sammamish, Washington 98074 (US); MULLINS, Nathan A., Highland Ranch, Colorado 80126 (US); MALININ, Yuriy, Edina, MN 55436 (US)
(74) Representative: Kramer Barske Schmidtchen Patentanwälte PartG mbB
(86) International application number: PCT/US2014/051517
(87) International publication number: WO 2015/026733

(56) References cited:
- US-A1- 2008 085 042
- US-A1- 2012 184 858
- US-A1- 2012 184 863
- US-B1- 6 368 285
- US-B2- 7 515 954
- VESSELA KRASTEVA ET AL: "QRS Template Matching for Recognition of Ventricular Ectopic Beats", ANNALS OF BIOMEDICAL ENGINEERING, KLUWER ACADEMIC PUBLISHERS-PLENUM PUBLISHERS, NE, vol. 35, no. 12, 1 September 2007 (2007-09-01), pages 2065-2076, XP019547587, ISSN: 1573-9686, DOI: 10.1007/S10439-007-9368-9
- GREENHUT S E ET AL: "Comparison of a new template matching algorithm, correlation, and area of difference methods for detection of ventricular tachycardia", COMPUTERS IN CARDIOLOGY 1992, PROCEEDINGS OF DURHAM, NC, USA 11-14 OCT. 1992, LOS ALAMITOS, CA, USA,IEEE COMPUT. SOC, US, 11 October 1992 (1992-10-11), pages 371-374, XP010032062, DOI: 10.1109/CIC.1992.269354 ISBN: 978-0-8186-3552-6
- Wikipedia: "Pearson correlation coefficient", , 27 April 2017 (2017-04-27), XP055368150, Retrieved from the Internet: URL:https://en.wikipedia.org/wiki/Pearson_ correlation_coefficient [retrieved on 2017-04-27]
- Wikipedia: "Cross-correlation", , 11 December 2017 (2017-12-11), XP055433814, Retrieved from the Internet: URL:https://en.wikipedia.org/wiki/Cross-co rrelation [retrieved on 2017-12-11]

## Description

### CROSS-REFERENCE TO RELATED APPLICATIONS

This application claims the benefit of United States provisional application no. 61/867,860, filed 20 August 2013.

### BACKGROUND

The instant disclosure relates to electrophysiological mapping, such as may be performed in cardiac diagnostic and therapeutic procedures. In particular, the instant disclosure relates to systems, apparatuses, and methods for generating an electrophysiology map from data collected by a roving electrophysiology probe.

Electrophysiological mapping, and more particularly electrocardiographic mapping, is a part of numerous cardiac diagnostic and therapeutic procedures. As the complexity of such procedures increases, however, the electrophysiology maps utilized must increase in quality, in density, and in the rapidity and ease with which they can be generated.

US 2012/0184863 A1 relates to the generation of an electroanatomical representation of a patient's heart based on signals measured at electrodes and information about the position of the electrodes.

US 2012/0184858 A1 relates to the determination and representation of physiology information relating to a heart surface.

VESSELA KRASTEVA ET AL: "QRS Template Matching for Recognition of Ventricular Ectopic Beats", ANNALS OF BIOMEDICAL ENGINEERING, KLUWER ACADEMIC PUBLISHERS-PLENUM PUBLISHERS, NE, vol. 35, no. 12, 1 September 2007 (2007-09-01), pages 2065-2076, XP019547587, ISSN: 1573-9686, DOI: 10.1007/S10439-007-9368-9 discloses QRS template matching for recognition of ventricular ectopic beats.

GREENHUT S E ET AL: "Comparison of a new template matching algorithm, correlation, and area of difference methods for detection of ventricular tachycardia", COMPUTERS IN CARDIOLOGY 1992, PROCEEDINGS OF DURHAM, NC, USA 11-14 OCT. 1992, LOS ALAMITOS, CA, USA, IEEE COMPUT. SOC, US, 11 October 1992 (1992-10-11), pages 371-374, XP010032062, DOI: 10.1109/CIC.1992.269354 ISBN: 978-0-8186-3552-6 discloses a time domain template matching morphology techniques for inclusion in implantable cardioverter defibrillators for the detection of ventricular arrhythmias from intraventricular electrograms.

### BRIEF SUMMARY

The invention relates to a system for generating an electrophysiology map according to claim 1. Preferred embodiments are defined in the dependent claims.

Disclosed herein is a method of generating an electrophysiology map of a portion of a patient's anatomy, including: defining a location-based electrophysiology data point inclusion criterion; defining a rhythm-based electrophysiology data point inclusion criterion; collecting an electrophysiology data point with an electrophysiology probe, wherein the electrophysiology data point is associated with location-based inclusion data and rhythm-based inclusion data; comparing the location-based inclusion data associated with the electrophysiology data point to the defined location-based inclusion criterion; comparing the rhythm-based inclusion data associated with the electrophysiology data point to the defined rhythm-based inclusion criterion; and adding the electrophysiology data point to the electrophysiology map when both the location-based inclusion data associated with the electrophysiology data point satisfies the location-based inclusion criterion and the rhythm-based inclusion data associated with the electrophysiology data point satisfies the rhythm-based inclusion criterion.

The location-based inclusion criterion can be selected from the group consisting of a velocity criterion, a distance moved criterion, a dwell time criterion, and a proximity criterion. For example, a velocity criterion can be defined such that the location-based inclusion data for the electrophysiology data point satisfies the velocity criterion when a velocity of the electrophysiology probe at a time the electrophysiology data point is collected is below a preset velocity threshold, such as about 10 mm/sec. As another example, a distance moved criterion can be defined such that the location-based inclusion data for the electrophysiology data point satisfies the distance moved criterion when a distance from a location of the electrophysiology probe at a time the electrophysiology data point is collected to a location of the electrophysiology probe at a time an electrophysiology data point was most recently added to the electrophysiology map is above a preset distance threshold, such as about 3 mm.

The rhythm-based inclusion criterion can be selected from the group consisting of a cycle length criterion and an EKG matching criterion. For example, a cycle length criterion can be defined such that the rhythm-based inclusion data for the electrophysiology data point satisfies the cycle length criterion when a cycle length for the electrophysiology data point is within a preset range about an initial cycle length value, such as plus-or-minus about 20 ms. As another example, an EKG matching criterion can be defined such that the rhythm-based inclusion data for the electrophysiology data point satisfies the EKG matching criterion when a matching score for an EKG signal at a time the electrophysiology data point is collected exceeds a preset matching score threshold, such as about 85%. Further, it is contemplated that the matching score can be calculated relative to a plurality of EKG signals for a template heartbeat, where the template heartbeat can correspond to an initial electrophysiology data point added to the electrophysiology map.

In examples, the location-based inclusion data and the rhythm-based inclusion data for the electrophysiology data point are displayed to a user. Moreover, feedback can be provided to a user when the electrophysiology data point is added to the electrophysiology map.

Also disclosed herein is a method of generating an electrophysiology map of a portion of a patient's anatomy, including: defining a template beat, the template beat including a plurality of template EKG signals, each of the plurality of template EKG signals corresponding to a respective one of a plurality of EKG leads; collecting an electrophysiology data point with an electrophysiology probe, wherein the electrophysiology data point is associated with a plurality of instantaneous EKG signals, each of the plurality of instantaneous EKG signals corresponding to a respective one of the plurality of EKG leads; comparing at least some of the instantaneous EKG signals to corresponding ones of the template EKG signals to calculate a matching score; and adding the electrophysiology data point to the electrophysiology map when the calculated matching score exceeds a preset matching score threshold, such as about 85%.

The template beat can be defined by selecting a subset of the plurality of template EKG signals. Thereafter, the selected subset of the plurality of template EKG signals can be compared to corresponding ones of the instantaneous EKG signals.

The comparison of at least some of the instantaneous EKG signals to corresponding ones of the template EKG signals to calculate a matching score can include: computing a template area; computing a distance between the at least some of the instantaneous EKG signals and corresponding ones of the template EKG signals; and dividing the computed distance by the computed template area.

The comparison of at least some of the instantaneous EKG signals to corresponding ones of the template EKG signals to calculate a matching score can also employ the Pearson Correlation Coefficient. For example, a score Scan be computed according to the equation S = P * f(r), where Pis the Pearson Correlation Coefficient of the template EKG signals and the instantaneous EKG signals, r is the ratio of amplitudes of the template EKG signals and the instantaneous EKG signals and is defined such that 0 :Sr :S 1, andfi:r) is a monotonically increasing function with output 0 :Sfi:r) :S 1.

Also disclosed is a method of generating an electrophysiology map of a portion of a patient's anatomy, includes: defining an electrophysiology data inclusion criterion; collecting an electrophysiology data point with an electrophysiology probe, wherein the electrophysiology data point includes location data, electrophysiology data, and inclusion data; adding a geometry point corresponding to the location data for the electrophysiology data point to the electrophysiology map; comparing the inclusion data associated with the electrophysiology data point to the defined inclusion criterion; and adding the electrophysiology data associated with the electrophysiology data point to the electrophysiology map when the inclusion data associated with the electrophysiology data point satisfies the inclusion criterion.
The electrophysiology data inclusion criterion can be selected from the group consisting of a velocity criterion, a distance moved criterion, a dwell time criterion, a proximity criterion, a cycle length criterion, an EKG matching criterion, and combinations thereof. In certain aspects, the electrophysiology data inclusion criterion includes a location-based inclusion criterion and a rhythm-based inclusion criterion.

According to another aspect disclosed herein, a system for generating an electrophysiology map of a portion of a patient's anatomy includes an inclusion processor and a mapping processor. The inclusion processor can be configured to: analyze location-based inclusion data and rhythm-based inclusion data associated with an electrophysiology data point to determine whether the location-based inclusion data and rhythm-based inclusion data respectively satisfy a location-based inclusion criterion and a rhythm-based inclusion criterion; and add the electrophysiology data point to the electrophysiology map when the location-based inclusion data and rhythm-based inclusion data respectively satisfy the location-based inclusion criterion and the rhythm-based inclusion criterion. The mapping processor is configured to generate a graphical representation of the electrophysiology map from a plurality of electrophysiology data points added to the electrophysiology map by the inclusion processor.

In yet a further aspect, a system for generating an electrophysiology map of a portion of a patient's anatomy includes a comparison processor and a mapping processor. The comparison processor is configured to: compare an instantaneous EKG signal to a template EKG signal; calculate a matching score indicative of a morphology match between the instantaneous EKG signal and the template EKG signal; and add an electrophysiology data point to the electrophysiology map when the matching score exceeds a preset matching score threshold. The mapping processor is configured to generate a graphical representation of the electrophysiology map from a plurality of electrophysiology data points added to the electrophysiology map by the comparison processor.

In still another aspect disclosed herein, a system for generating an electrophysiology map of a portion of a patient's anatomy includes an inclusion processor and a mapping processor. The inclusion processor is configured to: analyze inclusion data associated with an electrophysiology data point to determine whether the inclusion data satisfies an inclusion criterion; add a geometry point corresponding to location data associated with the electrophysiology data point to the electrophysiology map; and add the electrophysiology data point to the electrophysiology map when the inclusion data satisfies the inclusion criterion. The mapping processor is configured to generate a graphical representation of the electrophysiology map from a plurality of electrophysiology data points added to the electrophysiology map by the inclusion processor.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 is a schematic diagram of a localization system, such as may be used in an electrophysiology study.
Figure 2 depicts an exemplary catheter used in an electrophysiology study.
Figures 3 and 4 depict exemplary electrophysiology maps and illustrate various aspects of the present disclosure.
Figure 5 is a flowchart depicting representative steps that can be followed in a method of generating an electrophysiology map according to an embodiment disclosed herein.
Figure 6 is a flowchart depicting representative steps that can be followed to detect beats in an electrophysiological signal.
Figure 7 depicts a representative plot of the output of the - dV dt filtering algorithm for beat detection, aligned timewise with 12-lead EKG signals.
Figure 8 is a flowchart depicting representative steps of a morphology classification algorithm according to an embodiment disclosed herein.

### DETAILED DESCRIPTION

The present disclosure provides methods, apparatuses and systems for the creation of electrophysiology maps (e.g., electrocardiographic maps). For purposes of illustration, several exemplary embodiments will be described in detail herein in the context of a cardiac electrophysiology procedure. It is contemplated, however, that the methods, apparatuses, and systems described herein can be utilized in other contexts.

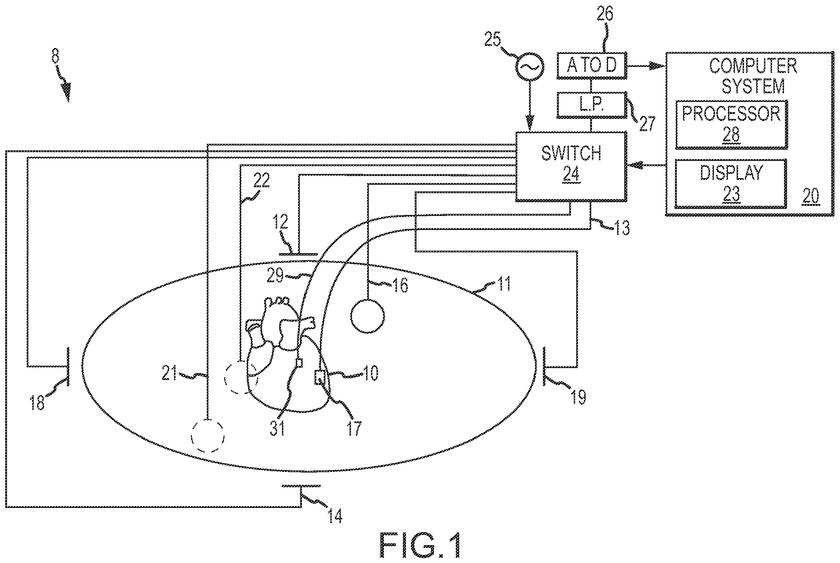

Figure 1 shows a schematic diagram of a localization system 8 for conducting cardiac electrophysiology studies by navigating a cardiac catheter and measuring electrical activity occurring in a heart 10 of a patient 11 and three-dimensionally mapping the electrical activity and/or information related to or representative of the electrical activity so measured. System 8 can be used, for example, to create an anatomical model of the patient's heart 10 using one or more electrodes. System 8 can also be used to measure electrophysiology data at a plurality of points along a cardiac surface and store the measured data in association with location information for each measurement point at which the electrophysiology data was measured, for example to create a diagnostic data map of the patient's heart 10.

As one of ordinary skill in the art will recognize, and as will be further described below, localization system 8 determines the location, and in some aspects the orientation, of objects, typically within a three-dimensional space, and expresses those locations as position information determined relative to at least one reference.

For simplicity of illustration, the patient 11 is depicted schematically as an oval. In the embodiment shown in Figure 1, three sets of surface electrodes (e.g., patch electrodes) are shown applied to a surface of the patient 11, defining three generally orthogonal axes, referred to herein as an x-axis, a y-axis, and a z-axis. In other embodiments the electrodes could be positioned in other arrangements, for example multiple electrodes on a particular body surface. As a further alternative, the electrodes do not need to be on the body surface, but could be positioned internally to the body.

In Figure 1, the x-axis surface electrodes 12, 14 are applied to the patient along a first axis, such as on the lateral sides of the thorax region of the patient (e.g., applied to the patient's skin underneath each arm) and may be referred to as the Left and Right electrodes. The y-axis electrodes 18, 19 are applied to the patient along a second axis generally orthogonal to the x-axis, such as along the inner thigh and neck regions of the patient, and may be referred to as the Left Leg and Neck electrodes. The z-axis electrodes 16, 22 are applied along a third axis generally orthogonal to both the x-axis and the y-axis, such as along the sternum and spine of the patient in the thorax region, and may be referred to as the Chest and Back electrodes. The heart 10 lies between these pairs of surface electrodes 12/14, 18/19, and 16/22.

An additional surface reference electrode (e.g., a "belly patch") 21 provides a reference and/or ground electrode for the system 8. The belly patch electrode 21 may be an alternative to a fixed intra-cardiac electrode 31, described in further detail below. It should also be appreciated that, in addition, the patient 11 may have most or all of the conventional electrocardiogram ("ECG" or "EKG") system leads in place. In certain embodiments, for example, a standard set of 12 ECG leads may be utilized for sensing electrocardiograms on the patient's heart 10. This ECG information is available to the system 8 (e.g., it can be provided as input to computer system 20). Insofar as ECG leads are well understood, and for the sake of clarity in the figures, the leads and their connections to computer system 20 are not illustrated in Fig. 1.

A representative catheter 13 having at least one electrode 1 7 (e.g., a distal electrode) is also shown. This representative catheter electrode 17 is referred to as the "roving electrode," "moving electrode," or "measurement electrode" throughout the specification. Typically, multiple electrodes on catheter 13, or on multiple such catheters, will be used. In one embodiment, for example, localization system 8 may comprise sixty-four electrodes on twelve catheters disposed within the heart and/or vasculature of the patient. Of course, this embodiment is merely exemplary, and any number of electrodes and catheters may be used within the scope of the present invention. Likewise, it should be understood that catheter 13 (or multiple such catheters) are typically introduced into the heart and/or vasculature of the patient via one or more introducers (not shown in Figure 1, but readily understood by the ordinarily skilled artisan).

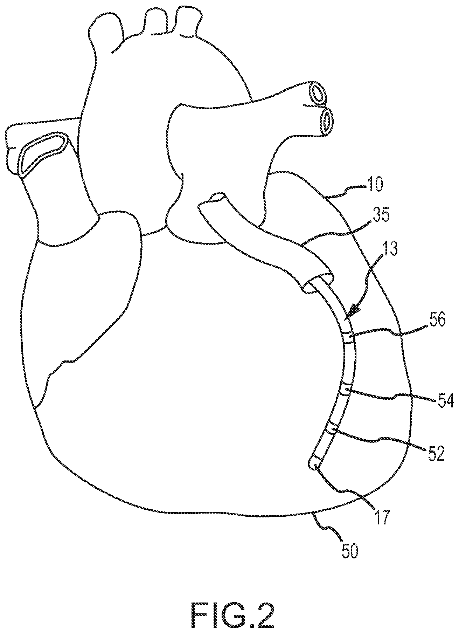

For purposes of this disclosure, a segment of an exemplary catheter 13 is shown in Figure 2. In Figure 2, catheter 13 extends into the left ventricle 50 of the patient's heart 10 through an introducer 35, the distal-most segment of which is shown in Figure 2. The construction of introducers, such as introducer 35, are well known and will be familiar to those of ordinary skill in the art, and need not be further described herein. Of course, catheter 13 can also be introduced into the heart 10 without the use of introducer 35.

Catheter 13 includes electrode 17 on its distal tip, as well as a plurality of additional measurement electrodes 52, 54, 56 spaced along its length in the illustrated embodiment. Typically, the spacing between adjacent electrodes will be known, though it should be understood that the electrodes may not be evenly spaced along catheter 13 or of equal size to each other. Since each of these electrodes 17, 52, 54, 56 lies within the patient, location data may be collected simultaneously for each of the electrodes by localization system 8.

Returning now to Figure 1, an optional fixed reference electrode 31 (e.g., attached to a wall of the heart 10) is shown on a second catheter 29. For calibration purposes, this electrode 31 may be stationary (e.g., attached to or near the wall of the heart) or disposed in a fixed spatial relationship with the roving electrodes (e.g., electrodes 17, 52, 54, 56), and thus may be referred to as a "navigational reference" or "local reference." The fixed reference electrode 31 may be used in addition or alternatively to the surface reference electrode 21 described above. In many instances, a coronary sinus electrode or other fixed electrode in the heart 10 can be used as a reference for measuring voltages and displacements; that is, as described below, fixed reference electrode 31 may define the origin of a coordinate system.

Each surface electrode is coupled to a multiplex switch 24, and the pairs of surface electrodes are selected by software running on a computer 20, which couples the surface electrodes to a signal generator 25. Alternately, switch 24 may be eliminated and multiple (e.g., three) instances of signal generator 25 may be provided, one for each measurement axis (that is, each surface electrode pairing).

The computer 20, for example, may comprise a conventional general-purpose computer, a special-purpose computer, a distributed computer, or any other type of computer. The computer 20 may comprise one or more processors 28, such as a single central processing unit (CPU), or a plurality of processing units, commonly referred to as a parallel processing environment, which may execute instructions to practice the various aspects of the present invention described herein.

Generally, three nominally orthogonal electric fields are generated by a series of driven and sensed electric dipoles (e.g., surface electrode pairs 12/14, 18/19, and 16/22) in order to realize catheter navigation in a biological conductor. Alternatively, these orthogonal fields can be decomposed and any pairs of surface electrodes can be driven as dipoles to provide effective electrode triangulation. Likewise, the electrodes 12, 14, 18, 19, 16, and 22 (or any number of electrodes) could be positioned in any other effective arrangement for driving a current to or sensing a current from an electrode in the heart. For example, multiple electrodes could be placed on the back, sides, and/or belly of patient 11. Additionally, such non-orthogonal methodologies add to the flexibility of the system. For any desired axis, the potentials measured across the roving electrodes resulting from a predetermined set of drive (source-sink) configurations may be combined algebraically to yield the same effective potential as would be obtained by simply driving a uniform current along the orthogonal axes.

Thus, any two of the surface electrodes 12, 14, 16, 18, 19, 22 may be selected as a dipole source and drain with respect to a ground reference, such as belly patch 21, while the unexcited electrodes measure voltage with respect to the ground reference. The roving electrodes 17, 52, 54, 56 placed in the heart 10 are exposed to the field from a current pulse and are measured with respect to ground, such as belly patch 21. In practice the catheters within the heart 10 may contain more or fewer electrodes than the four shown, and each electrode potential may be measured. As previously noted, at least one electrode may be fixed to the interior surface of the heart to form a fixed reference electrode 31, which is also measured with respect to ground, such as belly patch 21, and which may be defined as the origin of the coordinate system relative to which localization system 8 measures positions. Data sets from each of the surface electrodes, the internal electrodes, and the virtual electrodes may all be used to determine the location of the roving electrodes 17, 52, 54, 56 within heart 10.

The measured voltages may be used to determine the location in three-dimensional space of the electrodes inside the heart, such as roving electrodes 17, 52, 54, 56, relative to a reference location, such as reference electrode 31. That is, the voltages measured at reference electrode 31 may be used to define the origin of a coordinate system, while the voltages measured at roving electrodes 17, 52, 54, 56 may be used to express the location of roving electrodes 17, 52, 54, 56 relative to the origin. In some embodiments, the coordinate system is a three-dimensional (x, y, z) Cartesian coordinate system, although other coordinate systems, such as polar, spherical, and cylindrical coordinate systems, are contemplated.

As should be clear from the foregoing discussion, the data used to determine the location of the electrode(s) within the heart is measured while the surface electrode pairs impress an electric field on the heart. The electrode data may also be used to create a respiration compensation value used to improve the raw location data for the electrode locations as described in United States patent no. 7,263,397, which is hereby incorporated herein by reference in its entirety. The electrode data may also be used to compensate for changes in the impedance of the body of the patient as described, for example, in United States patent no. 7,885,707, which is also incorporated herein by reference in its entirety.

Therefore, in one representative embodiment, the system 8 first selects a set of surface electrodes and then drives them with current pulses. While the current pulses are being delivered, electrical activity, such as the voltages measured with at least one of the remaining surface electrodes and in vivo electrodes, is measured and stored. Compensation for artifacts, such as respiration and/or impedance shifting, may be performed as indicated above.

In some embodiments, the localization/mapping system is the EnSite™ Velocity™ cardiac mapping system of St. Jude Medical, Inc., which generates electrical fields as described above, or another localization system that relies upon electrical fields. Other localization systems, however, may be used in connection with the present teachings, including for example, the CARTO navigation and location system ofBiosense Webster, Inc., the AURORA® system of Northern Digital Inc., or Sterotaxis' NIOBE® Magnetic Navigation System, all of which utilize magnetic fields rather than electrical fields. The localization and mapping systems described in the following patents (all of which are hereby incorporated by reference in their entireties) can also be used with the present invention: United States Patent Nos. 6,990,370; 6,978,168; 6,947,785; 6,939,309; 6,728,562; 6,640,119; 5,983,126; and 5,697,377.

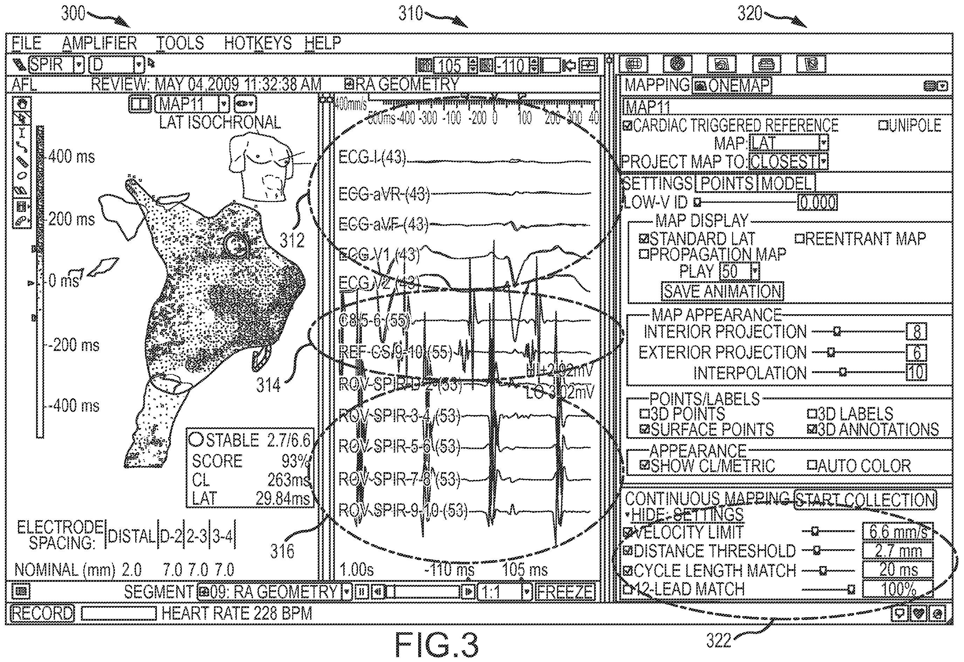

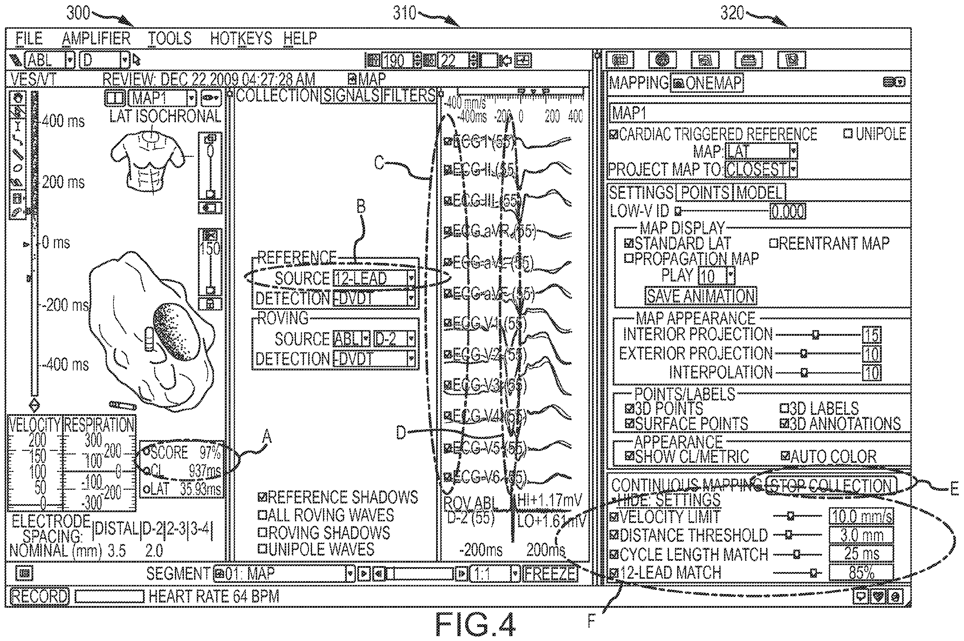

Figures 3 and 4 depict exemplary electrophysiology maps generated using various aspects disclosed herein and data collected and processed utilizing localization system 8 (e.g., using computer system 20). In general, those of ordinary skill in the art will be familiar with the content of Figures 3 and 4. Thus, the aspects thereof will only be described herein to the extent necessary to understand the instant disclosure.

Figures 3 and 4 each depict an exemplary interface, such as may be output on display 23, including, at the lower right hand comer ofleftmost panel 300, a "heads up" display (callout "A" in Figure 4). The "heads up" display provides feedback regarding the current status of certain inclusion criteria, which are described in detail below. More particularly, the "heads up" display provides information and visual cues (e.g., the use of red text to indicate that the current inclusion data does not satisfy the corresponding inclusion criterion) regarding the status of the inclusion criteria that are selected using the inclusion criterion control panel, shown at the bottom ofrightmost panel 320 (callout "F" in Figure 4). The "heads up" display and control panel can appear at other locations on the screen.

Figures 3 and 4 depict alternative configurations for center panel 310. In Figure 3, center panel 310 displays the signals from five EKG leads (e.g., white traces 312), from two reference electrodes (e.g., yellow traces 314), and from five roving electrodes (e.g., blue traces 316). In Figure 4, center panel 310 displays the signals from all twelve EKG leads. It also includes check boxes (callout "C") that can be used to enable or disable the signals from various leads for morphology comparison and/or classification purposes, as discussed in further detail below.

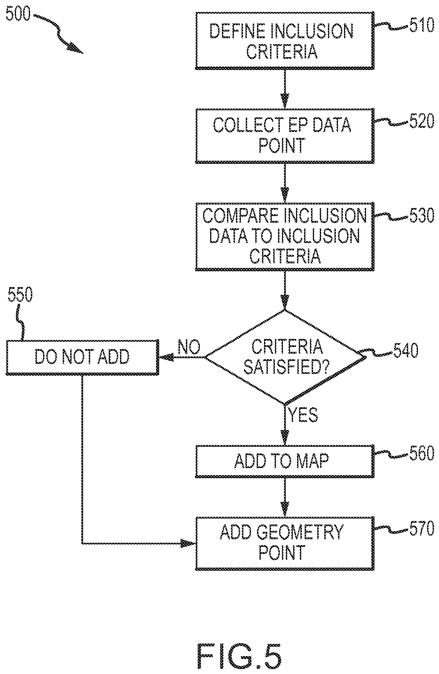

Figure 5 is a flowchart depicting representative steps of an exemplary method 500 for generating an electrophysiology map according to the instant disclosure. In block 510, one or more inclusion criteria are defined. Inclusion criteria can be generally classified as either "distance based" or "rhythm based," and, in some examples, at least one inclusion criterion of each type will be defined. In other examples, one inclusion criterion of one type will be defined. In still other examples, inclusion criteria may not be used at all, such that all electrophysiology data points are included in an electrophysiology map. Of course, other combinations are also contemplated.

As discussed further herein, exemplary inclusion criteria include catheter velocity (distance based), distance moved (distance based), proximity (distance based), dwell time (distance based), cycle length (rhythm based), and EKG match (rhythm based). Each of the foregoing will be discussed in further detail below. Other inclusion criteria can be utilized in addition to, or in lieu of, the foregoing inclusion criteria. For example, in some embodiments, respiration phase can be used in addition to catheter velocity, cycle length, and/or EKG match.

Any combination of inclusion criteria can be "active" at a given time (although the ordinarily skilled artisan will appreciate that certain combinations will be particularly desirable in specific applications, some of which are discussed in greater detail herein). As shown in Figures 3 and 4, the control panel in rightmost panel 320 includes check boxes to determine which inclusion criteria will be applied to a collected electrophysiology data point. Likewise, rightmost panel 320 also includes an interface (e.g., sliders 322) to adjust the inclusion criteria (e.g., to adjust the preset velocity threshold described below).

In step 520, an electrophysiology data point is collected, for example using one or more electrodes on catheter 13. As the ordinarily skilled artisan will appreciate, the electrophysiology data point includes both electrophysiology data and location data (e.g., information regarding the location of catheter 13 and/or the electrodes thereon, allowing the measured electrophysiology information to be associated with a particular location in space). It also includes (or is associated with) inclusion data (e.g., location-based inclusion data and/or rhythm-based inclusion data) that, as disclosed herein, can be used to determine whether or not the electrophysiology data point should be added to the electrophysiology map (or, in certain embodiments, which of several electrophysiology maps to which the electrophysiology data point belongs). This inclusion data can be displayed in the "heads up" display included in leftmost panel 300.

In step 530, the inclusion data for the collected electrophysiology data point is compared to the defined inclusion criteria. If the inclusion data for the collected electrophysiology data point does not satisfy the defined inclusion criteria (the "no" exit from decision block 540), then the electrophysiology data point is not added to the electrophysiology map (block 550). On the other hand, if the inclusion data for the collected electrophysiology data point does satisfy the defined inclusion criteria (the "yes" exit from decision block 540), then the electrophysiology data point is added to the electrophysiology map (block 560). The "heads up" display can provide visual cues to the user (e.g., by flashing and/or by displaying a green "go" icon for all active inclusion criteria) when the electrophysiology data point is added to the electrophysiology map.

Regardless of whether or not the inclusion data for the collected electrophysiology data point satisfies the defined inclusion criteria, a geometry point corresponding to the location data for the electrophysiology data point can optionally be added to the cardiac geometry model underlying the electrophysiology map (block 570). Of course, it is contemplated that inclusion criteria can also be employed to determine whether or not to add the location data to the cardiac geometry model underlying the electrophysiology map (that is, to treat block 570 as a decision block similar to decision block 540).

Each of the various inclusion criteria identified above offers certain advantages. For example, a catheter velocity criterion can help ensure that only electrophysiology data points collected when the probe (e.g., catheter 13) is relatively stable are included in the electrophysiology map. Thus, the catheter velocity criterion can be defined such that it is only satisfied when the electrophysiology data point is collected with the probe velocity below a preset velocity threshold (e.g., 10 mm/sec).

Similar to a velocity criterion, a dwell time criterion can help ensure that only electrophysiology data points collected when the probe (e.g., catheter 13) is relatively stable are included in the electrophysiology map. Thus, the dwell time criterion can be defined such that it is only satisfied if the probe (e.g., catheter 13) remains in a stable location for a preset threshold duration (e.g., between 1 sec and 8 sec, with the specific value typically chosen to match the selected segment length for purposes of complex fractionated electro gram ("CFE") analysis).

As another example, a distance moved criterion can help ensure that redundant points are excluded from the electrophysiology map. That is, a distance moved criterion helps ensure that the probe (e.g., catheter 13) is at a distinct location before adding a further electrophysiology data point to the electrophysiology map. In some examples, for example, the distance moved criterion can be defined such that it is only satisfied when the location of the probe when the electrophysiology data point is collected is at least a preset distance (e.g., 3 mm) from the location of the most recently added electrophysiology data point.

As yet another example, a proximity criterion can help ensure that the electrophysiology data point is sufficiently close to a geometry point. Thus, the proximity criterion can be defined such that it is only satisfied when the location of the probe when the electrophysiology data point is collected is within a preset distance (e.g., 4 mm) from a geometry point. It should be understood that the geometry points can be collected substantially simultaneously with the electrophysiology data points (e.g., in block 570 of Figure 5), in a separate procedure, or sourced from an external imaging modality (e.g., CT, MRI, or the like).

The rhythm-based inclusion criteria are utilized to help ensure that the rhythms match for all electrophysiology data points added to the electrophysiology map. Typically, the rhythm-based inclusion criteria compare a current beat (that is, the beat corresponding to the collected electrophysiology data point) to a template beat. The template beat can be the beat corresponding to the first electrophysiology data point added to the electrophysiology map, which can, for example, be manually captured in a conventional manner. Alternatively, the user can select the template beat from any beat recorded and/or stored in the electrophysiology map.

Cycle length criteria are desirable for use in atrial mapping (e.g., for mapping atrial tachycardia, fibrillation, and/or flutter). The application of cycle length criteria will compare the cycle length for the current beat to the cycle length for the template beat, and will generally be defined such that they are satisfied when the current beat cycle length is within a preset range (e.g., ± 20 ms) about the template beat cycle length. It is also contemplated to define a cycle length criterion that requires a preset number of beats (e.g., two consecutive beats) to fall within the preset range before a collected electrophysiology data point is added to the electrophysiology map. Of course, other variations are within the scope of the present teachings as well.

Cycle length criteria can be defined in numerous ways in accordance with the present teachings, including, without limitation: reference-to-reference; EKG-based QRS-to-QRS; EGM-to-reference; and EGM-to-EGM.

EKG match criteria are desirable for use in ventricular mapping (e.g., ventricular tachyarrhythmias), where it is beneficial for a practitioner to have a clear picture of what is happening for a given rhythm (the ordinarily skilled artisan will appreciate that each rhythm uses a different electrical propagation pattern through the heart). Unlike extant systems, which typically require the practitioner to "eyeball" when a current beat matches a template beat, EKG match criteria utilize morphology matching algorithms to compare the morphology of the current beat to the morphology of the template beat and assign a matching score that quantifies how well the current beat morphology matches the template beat morphology. If the matching score exceeds a preset matching score threshold (e.g., 85%), then the EKG matching criterion is satisfied and the collected electrophysiology data point can be added to the electrophysiology map.

Of course, each beat, whether template or current, includes a plurality of EKG signals, each of which corresponds to a respective EKG lead. It is contemplated that the EKG match criteria can be selectively applied to any or all of these leads, for example by selecting and de-selecting check boxes associated with each lead as seen in central panel 310 of Figure 4 (callout "C"). For example the practitioner can choose to exclude a particular EKG lead from the morphology matching algorithm because it has become disconnected, is exhibiting excessive noise, or for any other reason. Only the signals from the selected EKG leads will be subject to the EKG match criteria (that is, processed using the morphology matching algorithm).

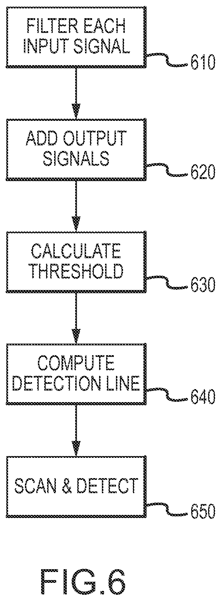

A first step in computing a matching score is to detect beats (e.g., the time(s) when R-waves are detected), for example in the signals from the selected EKG leads. The flowchart of Figure 6 depicts one representative series of steps that can be followed to detect beats from the selected EKG signals.

In step 610, each input signal (that is, the signal from each selected EKG lead) is filtered to produce an all-positive output signal that is designed to produce spikes for a particular wave feature. Suitable filtering algorithms include, for example, -dVdt, +dVdt, AbsDvdt, Min, Max, and AbsPeak, as discussed further herein.

Each of the foregoing filtering algorithms relies on the slope-amplitude product to help determine features of interest. Although a simple slope analysis could be computed by subtracting the value at time t1 from the value at time t2, where t1 and t2 are a fixed distance apart, this simple calculation does not differentiate between two time points with the same slope but different amplitudes. Thus, it is desirable to multiply the slope by the change in value for the same interval. Because the width of a feature is not fixed, the slope-amplitude product can be calculated multiple times for the same time point, each time with a different interval. The maximum slope-amplitude product can then be used for the filtering.

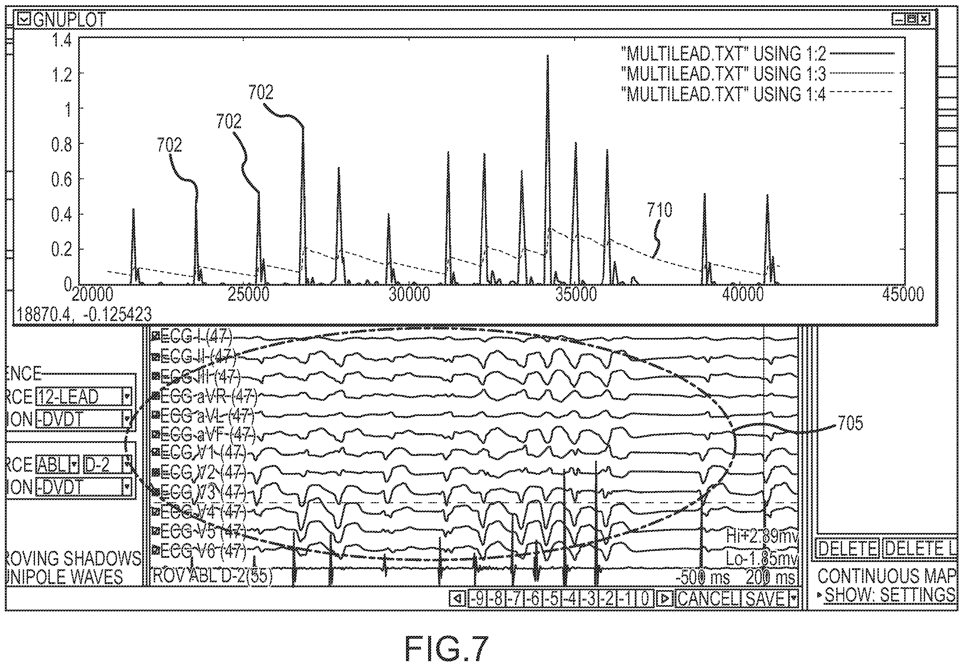

In the case of the - dV dt filtering algorithm, the slope-amplitude product is calculated for each time point. Intervals about this time point can range, for example, from 0 to ±25 ms for unipole signals and from 0 to± 12.5 ms for bipole signals. For positive slopes, the output is set to zero. In other cases, the output value is further modified to minimize features that are returning to baseline and to amplify features that are deviating from baseline. For example, the end value of the slope-amplitude interval can be analyzed and the output value set to zero if the end point value is positive (that is, returning to baseline) and multiplied by the square of the end point value in other cases (that is, deviating from baseline). This attenuates T waves and amplifies QRS complexes with large negative components. The square root of the best output value for a given time point can be taken in order to regularize the output. Figure 7 illustrates a plot of the output of the - dV dt algorithm, aligned timewise with the 12-lead EKG signals, in order to better illustrate the correlation between spikes 702 in the output of the - dV dt algorithm and the cardiac signals 705. The blue line 710 is the computed threshold value from the moving standard deviation, discussed further below.

The +dV dt filtering algorithm is similar to the - dV dt algorithm, except with inverted criteria.

For the AbsDvdt filtering algorithm, the +dV dt filtering algorithm and - dV dt filtering algorithm output values are calculated for each time point. The absolute maximum of the two values is output.

For the Min filtering algorithm, output zero for each time point where the sample value is positive. In all other cases, the slope-amplitude product is calculated separately for intervals before and after the current time point. Intervals are repeatedly analyzed, similar to the - dV dt filtering algorithm, until an optimal interval size is determined. The goal is to find a negative peak that has a strong downward slope-amplitude product before the current time point and a strong upward slope-amplitude product after the current time point. The slope-amplitude products from the intervals before and after the current time point are multiplied together. The square root of the result is then multiplied by the negative of the current value. The square root of the result is taken to regularize the output.

The Max filtering algorithm is similar to the Min filtering algorithm, except with inverted criteria.

The AbsPeak filtering algorithm outputs the absolute maximum of the Min and Max filtering algorithm outputs.

In step 620, the output signals from step 610 are added together.

In step 630, a moving threshold value for the summed output signal (e.g., the output of block 620) is calculated. The threshold value can be the square root of the moving variance (i.e., the moving standard deviation).

In step 640, a detection line is computed using the summed output signal (e.g., the output of block 620) and the moving standard deviation (e.g., the output of block 630).

In step 650, the output signal is forward scanned until the output signal exceeds the threshold. Upon locating such a point, forward scanning continues for a period of time. For EKG signals, this period is typically about 200 ms.

During this scanning period, the detection time is updated to represent the time of the maximum summed output signal (e.g., the output of block 620) during the scanning period. Once a detection time is chosen, there is a refractory period, typically of the same length as the above-described scanning period, from the time of the final detection before detection resumes for the next beat.

As an alternative or in addition to beat detection on EKG signals, beat detection can also be carried out on electrophysiology signals from intracardiac electrodes (e.g., roving electrodes 17, 52, 54, 56 on catheter 13). Beat detection methodologies for such signals, however, can differ from the above-described beat detection methodologies for EKG signals. In particular, intracardiac electrode beat detection methodologies can use a single signal and an analysis interval as an input, and can operate in a single pass that calculates an output value for every input value in the interval. The result is thus the time of the input value that creates the largest output value. Six different filtering algorithms, similar to those described above, can be utilized.

When applying the -dV dt filtering algorithm to an intracardiac roving electrode signal, the negative of the slope-amplitude product is calculated. If the result is negative, the output is zero. If not, the interval is varied and the maximum value from all intervals is output. For unipole signals, the time interval can be varied, for example, between± 25 ms. For bipole signals, the time interval can be varied, for example, between ± 10 ms. The application of the +dV dt filtering algorithm to an intracardiac roving electrode signal is similar, except the criteria are inverted. Likewise, the AbsDvDt filtering algorithm, when applied to an intracardiac roving electrode signal, outputs the time that had the greatest intermediate output value as between the -dV dt and +dV dt filtering algorithms.

The Min filtering algorithm, when applied to an intracardiac roving electrode signal, returns the time of the minimum sample value in the interval. The Max filtering algorithm, when applied to an intracardiac roving electrode signal, returns the time of the maximum sample value in the interval. The AbsPeak filtering algorithm, when applied to an intracardiac roving electrode signal, returns the time of the maximum absolute sample value in the interval.

No refractory period need be used for intracardiac roving electrode signals. In the event an intracardiac reference electrode signal is used, however, such as a signal from reference electrode 31, the refractory period can be set to about 120 ms, rather than the 200 ms refractory period utilized in beat detection based on EKG signals.

Following beat detection, a window is built around the detected R-wave, with the detected peak in the center of the window. Next, a distance function dis defined. The distance function d generates a distance between two waveforms containing a peak, such as the current beat waveform and the template beat waveform.

One suitable way to define the distance function dis to use a Dynamic Time Warping ("DTW") distance between the two waves. The ordinarily skilled artisan will appreciate how to apply a DTW algorithm in accordance with the teachings herein. One advantage of this method is that it does not require phase alignment between the two waveforms being compared. On the other hand, it results in increased computational complexity.

Alternatively, the distance function d can be defined as the Euclidean distance between the waveforms. When Euclidean distance is used, the waves can be shifted with respect to each other in steps from ±MaxStep in intervals of one. The MaxStep parameter can be selected to be the half-width of the waveform window, or, alternatively, a fixed value of between 1 and 5 samples for a sample rate of approximately 250 Hz. The distance function d can be set to the minimum distance between the waveforms after the shifting process.

In multi-lead analysis, the distances between a pair of beats can be computed separately for each EKG lead, and then linearly combined into a single distance measure. The linear combination can, for example, be the average of the waveforms used for the peak detection, or another suitable weighted combination of the individual leads' distances as defined by the user.

It is also contemplated that the beats (e.g., the template beat and the beat of interest) can be normalized, for example by subtracting the mean of each from themselves, before computing the distance function d.

In one aspect, the matching score for a particular beat is calculated as follows. First, for each template (that is, for the template beat from each selected EKG lead), a distance is computed between the template and a zero signal (that is, the model signal, for which all samples are set to zero). This distance is referred herein to as the "template area."

Then, for each waveform (e.g., for the current beat from each selected EKG lead), a distance is computed between the waveform and the template beat. This difference is divided by the template area, and the resulting ratio is subtracted from one and expressed as a percentage. If, however, the ratio is greater than one, a 0% matching score is assigned.

As discussed above, for multi-lead comparisons, the template area can be taken as the weighted sum of individual leads' template areas. Likewise, the distance between the current waveform and the template waveform can be taken as the weighted sum of individual leads' distances.

In addition to computing a matching score, beats can also be classified according to their morphologies into one or more different morphology maps. Indeed, the distance function d described above can also be used to select a particular waveform morphology for a diagnostic map and/or for morphology classification purposes. In other words, in addition to determining how well a current beat matches a single template beat for generation of a single map, the distance function d can be used to determine to which of several template beats a current beat best matches.

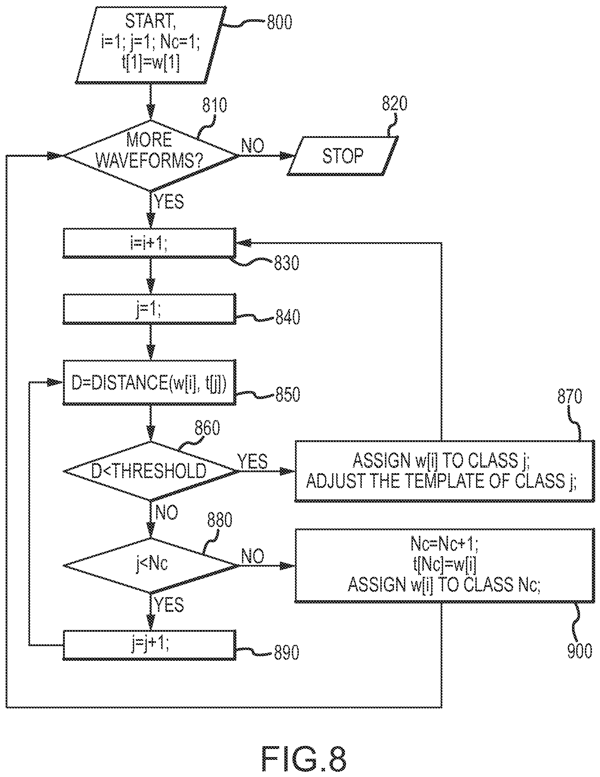

Figure 8 is a flowchart depicting representative steps that can be followed in a morphology classification algorithm according to an aspect disclosed herein, which starts at block 800. Decision block 810 examines whether there are additional waveforms (that is, beats) to be classified. If not, the process ends in block 820. If so, the process proceeds to block 830 for analysis of the ith waveform against the /h template (block 840).

In block 850, the distance function dis computed for the zth waveform vis-a-vis the /h template. Decision block 860 examines whether d is less than a preset threshold. If so, then it can be concluded that the ith waveform is a suitable match to the /h template, and can be classified accordingly in block 870.

If dis not less than the preset threshold, and there are remaining templates (block 880), the zth waveform is tested in similar fashion against the remaining templates (block 890). If the zth template does not suitably match any of the j templates, however, then a new morphology class is created and a new template added in block 900. The process iterates until all i waveforms are either classified or used to create new templates.

In certain embodiments, the Pearson Correlation Coefficient can be used in addition to the distance function din order to compute a matching score and/or classify beats by morphology. For example, a score Scan be computed according to the equation S = P * f (r), where Pis the Pearson Correlation Coefficient of the template beat and the current beat under consideration, r is the ratio of amplitudes of the template beat and the current beat under consideration, and is defined such that 0 :Sr :S 1 (the larger amplitude is in the denominator when computing r), and fir) is a monotonically increasing function with output in the range 0 :S fir) :S 1. The amplitudes can be measured by the standard deviation or peak-to-peak measurement.

Although several embodiments of this invention have been described above with a certain degree of particularity, those skilled in the art could make numerous alterations to the disclosed embodiments without departing from the spirit or scope of this invention.

For example, although the description above refers to data collected by only a single electrode, it is contemplated that multiple electrodes (e.g., 17, 52, 54, 56) can be utilized simultaneously.

As another example, the electrophysiology map generated in accordance with the present teaching can be augmented with manually-collected electrophysiology data points.

As still another example, additional inclusion criteria can be applied as a filter to cull points from the electrophysiology map. That is, once points are collected with certain inclusion criteria active (e.g., velocity and cycle length), one or more additional inclusion criteria (e.g., proximity) can be applied to cull points from the electrophysiology map, for example to rule out points that are interior to the geometry model.

As yet another example, EKG matching criterion can also employ Normalized Cross-Correlation in order to compute the matching score.

As a further example, in addition to the distance based and rhythm based inclusion criteria discussed above, force based inclusion criteria (e.g., a measure of how hard catheter 13 is pressing into adjacent tissue) and/or electrical coupling based inclusion criteria (e.g., the Electrical Coupling Index ("ECI") as discussed in United States patent no. 8,449,535, which is hereby incorporated herein in its entirety) can be defined and employed in analogous manner to the teachings herein.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the spirit of the invention as defined in the appended claims.

## Claims

1. A system for generating an electrophysiology map of a portion of a patient's anatomy, comprising:
a comparison processor configured to:
compare two or more instantaneous EKG signals to corresponding ones of template EKG signals;
calculate a matching score indicative of a morphology match between the two or more instantaneous EKG signals and the corresponding ones of the template EKG signals; and
add an electrophysiology data point to the electrophysiology map when the matching score exceeds a preset matching score threshold; and
a mapping processor configured to generate a graphical representation of the electrophysiology map from a plurality of electrophysiology data points added to the electrophysiology map by the comparison processor, **characterized in that** the matching score is calculated using a ratio between a distance between the two or more instantaneous EKG signals and the corresponding ones of the template EKG signals and a template area,
wherein the template area is computed by computing a distance between each of the two or more template EKG signals and a zero signal, which is a model signal for which all samples are set to zero, and
wherein the template area is computed as a weighted sum of distances between each of the two or more template EKG signals and the zero signal.

2. The system according to claim 1, wherein the method of calculating the matching score further comprises:
expressing the quotient of the computed distance and the computed template area as a percentage;
setting the matching score to zero if the percentage is greater than 100%; and
setting the matching score equal to the difference between 100% and the percentage otherwise.

3. The system according to claim 1, wherein the preset matching score threshold is 85%.

4. The system according to claim 1, wherein a further matching score is computed according to an equation *S* = *P* * *f(r),* where *P* is the Pearson Correlation Coefficient of the one or more template EKG signals, *r* is the ratio of amplitudes of the one or more template EKG signals and the one or more instantaneous EKG signals and is defined such that 0 ≤ *r* ≤ 1, and *f(r)* is a monotonically increasing function with output 0 ≤ *f(r)* ≤ 1.

## Patentansprüche

1. System zum Erzeugen einer elektrophysiologischen Karte eines Bereichs der Anatomie eines Patienten, mit
einem Vergleichsprozessor, der konfiguriert ist zum:
Vergleichen von zwei oder mehreren augenblicklichen EKG Signalen mit entsprechenden EKG Mustersignalen;
Berechnen einer Übereinstimmungsbewertung, die eine morphologische Übereinstimmung zwischen zwei oder mehreren augenblicklichen EKG Signalen und den entsprechenden der EKG Mustersignalen angibt; und
Hinzufügen eines elektrophysiologischen Datenpunkts zu der elektrophysiologischen Karte, wenn die Übereinstimmungsbewertung einen voreingestellten Übereinstimmungsbewertungs-Schwellenwert überschreitet; und
einem Abbildungsprozessor, der konfiguriert ist zum Erzeugen einer grafischen Darstellung der elektrophysiologischen Karte von einer Mehrzahl von elektrophysiologischen Datenpunkten, die zu der elektrophysiologischen Karte durch den Vergleichsprozessor hinzugefügt worden sind, **dadurch gekennzeichnet, dass**
die Übereinstimmungsbewertung berechnet wird, indem ein Verhältnis verwendet wird zwischen einem Abstand der zwei oder mehreren augenblicklichen EKG Signalen und den entsprechenden der EKG Mustersignalen und einem Musterbereich, wobei
der Musterbereich berechnet wird, indem ein Abstand zwischen jedem der zwei oder der mehreren EKG Mustersignalen und einem Nullsignal berechnet wird, welches ein Modellsignal ist, für das alle Muster auf null gesetzt sind, und
wobei der Musterbereich errechnet wird als eine gewichtete Summe von Abständen zwischen jedem von den zwei oder den mehreren EKG Mustersignalen und dem Nullsignal.

2. System nach Anspruch 1, bei dem das Verfahren zum Berechnen der Übereinstimmungsbewertung ferner aufweist:
Ausdrücken des Quotienten des berechneten Abstands und des berechneten Musterbereichs in Prozent;
Einstellen der Übereinstimmungsbewertung auf null, wenn der Prozentsatz größer als 100% ist; und
Einstellen der Übereinstimmungsbewertung auf die Differenz zwischen 100% und einem anderweitigen Prozentsatz.

3. System nach Anspruch 1, bei dem der voreingestellte Übereinstimmungsbewertungs-Schwellenwert 85% ist.

4. System nach Anspruch 1, bei dem ferner die Übereinstimmungsbewertung berechnet wird gemäß einer Gleichung *S* = *P* * *f(r),* wobei *P* der Pearson Correlation Coefficient von einem oder von mehreren EKG Mustersignalen ist, *r* das Verhältnis der Amplituden des einen oder der mehreren EKG Mustersignalen und dem einen oder den mehreren augenblicklichen EKG Signalen und derart definiert ist, dass *0* ≤ *r* ≤ *1* ist, und *f(r)* eine monoton ansteigende Funktion mit einer Ausgabe *0* ≤ *f(r)* ≤ *1* ist.

## Revendications

1. Système pour générer une carte d'électrophysiologie d'une partie d'anatomie d'un patient, comprenant :
un processeur de comparaison configuré pour :
comparer deux signaux ECG instantanés ou plus aux signaux correspondant des signaux ECG modèles ;
calculer un score de correspondance indiquant une correspondance morphologique entre les deux signaux ECG instantanés ou plus et les signaux correspondants des signaux ECG modèles ; et
ajouter un point de données d'électrophysiologie à la carte d'électrophysiologie lorsque le score de correspondance dépasse un seuil de score de correspondance prédéfini ; et
un processeur de cartographie configuré pour générer une représentation graphique de la carte d'électrophysiologie à partir d'une pluralité de points de données d'électrophysiologie ajoutés à la carte d'électrophysiologie par le processeur de comparaison, **caractérisé par le fait que**
le score de correspondance est calculé en utilisant un rapport entre une distance entre les deux signaux ECG instantanés ou plus et les signaux correspondant des signaux ECG modèles et un zone modèle,
dans lequel la zone modèle est calculée en calculant une distance entre chacun des deux signaux ECG modèles ou plus et un signal nul, qui est un signal modèle pour lequel tous les échantillons sont mis à zéro, et
dans lequel la zone modèle est calculée sous la forme d'une somme pondérée de distances entre chacun des deux signaux ECG modèles ou plus et du signal nul.

2. Système selon la revendication 1, dans lequel le procédé de calcul du score de correspondance comprend en outre le fait :
d'exprimer le quotient de la distance calculée et de la zone modèle calculée en pourcentage ;
de définir le score de correspondance à zéro si le pourcentage est supérieur à 100% ; et
de définir le score de correspondance égal à la différence entre 100% et le pourcentage autrement.

3. Système selon la revendication 1, dans lequel le seuil de score de correspondance prédéfini est de 85%.

4. Système selon la revendication 1, dans lequel un score de correspondance additionnel est calculé selon une équation *S* = *P* * *f(r),* où *P* est le Coefficient de Corrélation de Pearson du ou des signaux ECG modèles, *r* est le rapport d'amplitudes du ou des signaux ECG modèles et du ou des signaux ECG instantanés et est défini de sorte que 0 ≤ *r* ≤ 1, et *f(r)* est une fonction monotone croissante avec pour sortie 0 ≤ *f(r)* ≤ 1
